# EUROPEAN PATENT APPLICATION

(11) **EP 4 796 064 A2**
(43) Date of publication of application: **26.08.2026**
(21) Application number: 24879030.5
(22) Date of filing: 16.10.2024
(51) Int. Cl.: A61B 34/20

(54) **OCT IMAGING-BASED POSITIONING AND NAVIGATION METHOD FOR ORTHOPEDIC SURGICAL ROBOT**

(30) Priority: 16.10.2023 CN 202311338474
(71) Applicant: CHUNFENGHUAYU (SUZHOU) INTELLIGENT MEDICAL TECHNOLOGY CO., LTD., Taicang Suzhou, Jiangsu 215413 (CN)
(72) Inventor: YOUNG, Victor, Suzhou, Jiangsu 215413 (CN); CHEN, Chaoliang, Suzhou, Jiangsu 215413 (CN); SHI, Weisong, Suzhou, Jiangsu 215413 (CN)
(74) Representative: Maiwald GmbH
(86) International application number: PCT/CN2024/125218
(87) International publication number: WO 2025/082389

(57) **Abstract**

The present disclosure relates to an OCT imaging-based positioning and navigation method for an orthopedic surgical robot, which includes the following steps: S1: scanning, by the OCT imaging probe, a region to be operated on to acquire an OCT image of the region to be operated on; S2: performing a first registration between the OCT image of the region to be operated on and a preoperative medical image to acquire preoperative planning information; S3: scanning, by the OCT imaging probe, the surgical tool robotic arm or the surgical tool to acquire a fine registration matrix for registration between the imaging device robotic arm and the surgical tool robotic arm; S4: scanning, by the OCT imaging probe, the region to be operated on in real time to update a positional relationship between the region to be operated on and the imaging device robotic arm; S5: updating, in real time by the fine registration matrix, real-time coordinates of the surgical tool robotic arm in an OCT coordinate system and a real-time pose of the surgical tool in the OCT coordinate system; and S6: controlling, based on the real-time coordinates and the real-time pose and in accordance with registered surgical planning information, the surgical tool robotic arm and/or the surgical tool to perform surgical operations.

## Description

For all purposes, the present disclosure claims priority to Chinese Patent Application No. 202311338474.2 filed on October 16, 2023, the entire content of which is incorporated herein by reference as part of the present disclosure.

### TECHNICAL FIELD

The present disclosure relates to an OCT imaging-based positioning and navigation method for an orthopedic surgical robot, the orthopedic surgical robot including an OCT imaging device, a surgical tool robotic arm for mounting surgical tools, and an imaging device robotic arm for mounting the OCT imaging device.

### BACKGROUND

Infrared navigation is a positioning and navigation technology that locates various surgical instruments and surgical areas by detecting reflective balls. To achieve precise intraoperative navigation, an infrared positioning and navigation system usually needs to be registered with intraoperative three-dimensional images, intraoperative CT, and preoperative CT before starting to navigate the surgical robot. This easily exposes both surgeons and patients to a large amount of radiation during surgery. Meanwhile, steps such as pushing in and pulling out the intraoperative three-dimensional CT machine for scanning complicate the surgical procedure, and patient movement, breathing, or other intraoperative motions cannot be detected in a timely manner, which reduces navigation accuracy and affects the final surgical outcome. Optical Coherence Tomography (OCT) is a type of optical tomography technology that can provide real-time two-dimensional or three-dimensional images with micron-level resolution and millimeter-level penetration depth. Owing to its advantages of providing depth information, non-invasiveness, fast imaging, and high resolution, OCT is widely used for intraoperative navigation in ophthalmic surgery.

### SUMMARY

The technical problem to be solved by the present disclosure is to provide an OCT imaging-based positioning and navigation method for an orthopedic surgical robot, which can significantly reduce the radiation exposure received by patients during surgery.

This technical problem is solved by an OCT imaging-based positioning and navigation method for an orthopedic surgical robot. The orthopedic surgical robot includes an OCT imaging device with an OCT imaging probe, a surgical tool robotic arm for mounting surgical tools, and an imaging device robotic arm for mounting the OCT imaging device. According to the present disclosure, the positioning and navigation method includes the following steps:
S1: scanning, by the OCT imaging probe, a region to be operated on to acquire an OCT image of the region to be operated on;
S2: performing a first registration between the OCT image and a preoperative medical image to acquire preoperative planning information;
S3: scanning, by the OCT imaging probe, the surgical tool robotic arm or the surgical tool to acquire a fine registration matrix for registration between the imaging device robotic arm and the surgical tool robotic arm;
S4: scanning, by the OCT imaging probe, the region to be operated on in real time to update a positional relationship between the region to be operated on and the imaging device robotic arm;
S5: updating, in real time by the fine registration matrix, real-time coordinates of the surgical tool robotic arm in an OCT coordinate system and a real-time pose of the surgical tool in the OCT coordinate system; and
S6: controlling, based on the real-time coordinates and the real-time pose and in accordance with registered surgical planning information, the surgical tool robotic arm and/or the surgical tool to perform surgical operations. Because the method according to the present disclosure eliminates intraoperative scanning on the patient by medical imaging device, it greatly reduces the radiation exposure received by the patient and is more conducive to the patient's physical health.

In an extended embodiment according to the present disclosure, in the step S2, the surgical planning information in the preoperative medical image is converted into surgical planning information in the OCT coordinate system by a first registration matrix, where the first registration matrix is calculated based on a three-dimensional coordinate point cloud of the OCT image and a three-dimensional coordinate point cloud of the medical image.

In a further extended embodiment according to the present disclosure, before the step S3, a second registration matrix between the imaging device robotic arm and the surgical tool robotic arm is calculated based on a positional relationship between the imaging device robotic arm and the surgical tool robotic arm, where the second registration matrix is calculated based on coordinates of the imaging device robotic arm in a robot coordinate system and coordinates of the surgical tool robotic arm in the robot coordinate system.

In a still further extended embodiment according to the present disclosure, coordinates of visual field region of the OCT imaging probe in an imaging device robotic arm coordinate system are transformed into coordinates in a surgical tool robotic arm coordinate system by the second registration matrix, so as to move the surgical tool robotic arm into the visual field region, where the visual field region is preferably an area near the region to be operated on that is scanned by the OCT imaging device.

In a further extended embodiment according to the present disclosure, the fine registration matrix is acquired by scanning a first marker that is provided on the surgical tool robotic arm or the surgical tool and is recognizable by the OCT imaging probe.

In a still further extended embodiment according to the present disclosure, in the step S4, a second marker that is provided in the region to be operated on and is recognizable by the OCT imaging probe is scanned by the OCT imaging probe to acquire real-time coordinates of the region to be operated on in the OCT coordinate system. This avoids errors in surgical planning caused by inevitable slight movements of the patient or the operating table.

In a further extended embodiment according to the present disclosure, in the step S5, real-time coordinates of the surgical tool robotic arm in a surgical tool robotic arm coordinate system are acquired by the surgical tool robotic arm system itself, and a real-time pose of the surgical tool in the surgical tool robotic arm coordinate system is acquired based on a size of the surgical tool and a shape of the surgical tool; and, the real-time coordinates and the real-time pose are respectively transformed into the real-time coordinates of the surgical tool robotic arm in the OCT coordinate system and the real-time pose of the surgical tool in the OCT coordinate system by the fine registration matrix.

In a further extended embodiment according to the present disclosure, a capturing apparatus with a same visual field as the OCT imaging probe is provided, and a marker for marking the region to be operated on that is recognizable by the capturing apparatus is identified by the capturing apparatus to acquire original position coordinates of the region to be operated on in the OCT imaging device coordinate system, so as to calculate an imaging position directly above and facing the region to be operated on based on the original position coordinates, and move the imaging device robotic arm to the imaging position. This enables automatic movement of the imaging device robotic arm to the imaging position and improves the automation degree of the orthopedic surgical robot.

In a further extended embodiment according to the present disclosure, the preoperative medical image is acquired by medical imaging device, and the surgical planning information is recorded on a three-dimensional model of the preoperative medical image.

### BRIEF DESCRIPTION OF DRAWINGS

The features and advantages of the present disclosure are further explained below with reference to the accompanying drawings, in which:
Fig. 1 is a non-limiting embodiment of an orthopedic surgical robot capable of performing an OCT imaging-based positioning and navigation method; and
Fig. 2 is a flow chart of an OCT imaging-based positioning and navigation method for an orthopedic surgical robot according to the present disclosure.

With reference to the accompanying drawings, the same reference numerals refer to the same components in multiple views. The drawings mainly illustrate the positional, connection, mating, and dimensional relationships between various elements (e.g., components and parts), but are not intended to limit the dimensions and specific implementation forms of these elements.

### DETAILED DESCRIPTION

The term "registration matrix" according to the present disclosure shall be understood as a matrix for transforming coordinates under two respective coordinate systems, e.g., coordinate system A and coordinate system B. The term "pose" according to the present disclosure shall be understood as a position and an orientation in the same coordinate system.

It should be understood that the present disclosure may adopt various alternative changes and step sequences unless explicitly specified otherwise. It should also be understood that the specific equipment and processes shown in the drawings and described in the following specification are merely exemplary and non-limiting embodiments or aspects.

The technical term "orthopedic surgery" in the present disclosure includes, but is not limited to, laminectomy, discectomy, vertebroplasty, lumbar fusion, and the like. The ordinal numbers "first" and "second" only indicate different components and do not imply a limitation on the order.

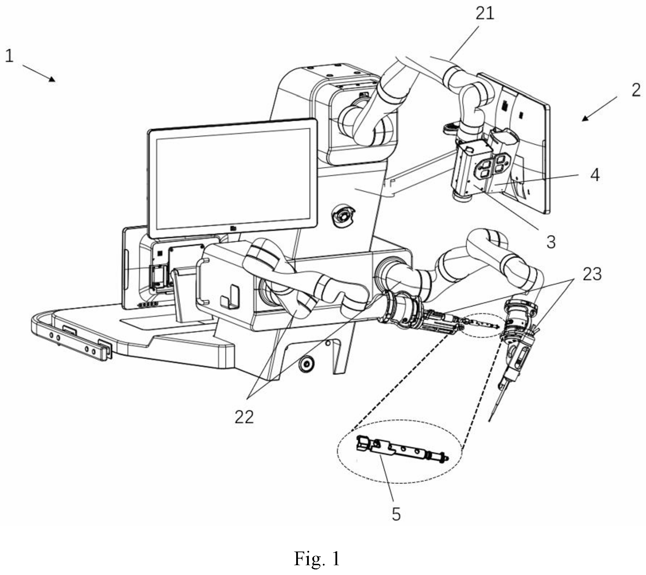

Fig. 1 shows a non-limiting embodiment of an orthopedic surgical robot 1 for performing an OCT imaging-based positioning and navigation method according to the present disclosure. Only the main mechanical components related to performing the OCT imaging-based positioning and navigation method are shown in this figure. The orthopedic surgical robot 1 according to the present disclosure mainly includes a robotic arm device 2, an OCT imaging-based positioning and navigation device (only the execution component of its imaging device, i.e., an OCT imaging probe 3, is shown in this figure), and a control device (not shown) for controlling the movement of the robotic arm device 2 and the positioning and navigation device. The orthopedic surgical robot 1 generally also has a cart device (not shown) for carrying the robotic arm device 2.

In a non-limiting embodiment of the present disclosure, the robotic arm device 2 includes an imaging device robotic arm 21 for mounting the imaging device and at least one surgical tool robotic arm 22 for mounting surgical tools 23, where each robotic arm is preferably implemented as a multi-axis robotic arm as shown in Fig. 1. The imaging device includes an OCT imaging probe 3 for OCT imaging-based positioning and navigation and a capturing apparatus 4 for providing visualization of the surgical area for surgeons and nurses. In this embodiment, the OCT imaging probe 3 and the capturing apparatus 4 are arranged at a free end of the imaging device robotic arm 21 such that they have the same visual field and are communicatively connected to the control device (not shown) to keep the capturing apparatus in a working position and orientation throughout the surgical procedure. The surgical tools 23 for performing orthopedic surgery can be detachably mounted at a free end of the surgical tool robotic arm 22 close to the surgical area, respectively, and the OCT imaging probe 3 and the capturing apparatus 4 can be fixedly mounted at the free end of the imaging device robotic arm 21. The robotic arm device 2 can also serve as a mechanical driver for the corresponding surgical tools 23, the OCT imaging probe 3, and the capturing apparatus 4, respectively. To avoid medical staff replacing the surgical tools 23 back and forth during the surgical procedure and thereby improving the automation degree of the surgical robot 1, the robotic arm device 2 in this embodiment preferably includes one imaging device robotic arm and two surgical tool robotic arms 22.

The surgical tools 23 respectively mounted on the surgical tool robotic arms 22 can be implemented either as surgical tools for surgical operations, such as osteotomes, screw drivers, drills, retractors, bone forceps, bone mallets, or other types of surgical tools, or as implant inserters for placing implants, such as screw drivers. In a non-limiting embodiment of the present disclosure, one of the surgical tools 23 can be designed as a first implant inserter, e.g., a first screw placement tool, for placing a first implant, e.g., a first screw, into the body, and another surgical tool 23 can be optionally designed as a surgical tool in the form of a drilling tool for drilling or as a second implant inserter, e.g., a second screw placement tool, for placing a second implant, e.g., a second screw, into the body.

As shown in the dashed circle in Fig. 1, a first marker 5 for fine registration that is recognizable by the OCT imaging probe 3 may be arranged on one of the surgical tools 23.

Certainly, depending on the complexity of the surgical situation, the number of surgical tool robotic arms 22 may be designed to be more than two that work in coordination with each other to complete the surgery more efficiently. Conversely, to reduce the load of data analysis and processing of the control device, the number of surgical tool robotic arms 22 may also be designed as only one multifunctional surgical tool robotic arm that can, for example, in spinal surgery, mount both a drill for drilling and a screw placement tool for screwing. An interface for connecting different surgical tools 23 is arranged at the free end of this single multifunctional surgical tool robotic arm. Different from the surgical robot 1 with two surgical tool robotic arms, this orthopedic surgical robot 1 with only one surgical tool robotic arm requires medical staff to participate in replacing the surgical tools 23 during surgical operations. Therefore, the automation degree of the surgical robot 1 in performing surgery with only one surgical tool robotic arm is lower than that with two surgical tool robotic arms.

The surgical tool robotic arm 22 positions and orients the corresponding surgical tool 23 of the orthopedic surgical robot 1 according to commands issued by a surgical planning module and a tracking device for the orthopedic surgical robot 1 to automatically complete at least two types of operational functions in orthopedic surgery, such as automated drilling and automatic screw placement. The surgical operational functions may also include osteotomy, joint replacement, bone traction, debridement, insertion and removal of Kirschner wires, or other surgical operational functions. For example, to realize functions such as drilling and screw placement, the surgical tool robotic arm 22 first, based on trajectories for drilling and/or screw placement obtained from preoperative planning, performs the operation of the surgical tool robotic arm 22 with real-time monitoring and guidance by the OCT positioning and navigation system, thereby ensuring precise control of the surgical tool robotic arm 22 to complete drilling and/or screw placement. In particular, in a non-limiting embodiment of the present disclosure, precise control of screw placement by one surgical tool robotic arm 22 and sequential drilling and screw placement by the other surgical tool robotic arm 22 is ensured.

The control device is communicatively coupled with the robotic arm device 2 and the OCT imaging-based positioning and navigation device, and mainly includes a data analysis and processing module, a surgical planning module, and a robotic arm control module, where the control device may be implemented as a computer device with a computer-readable storage medium.

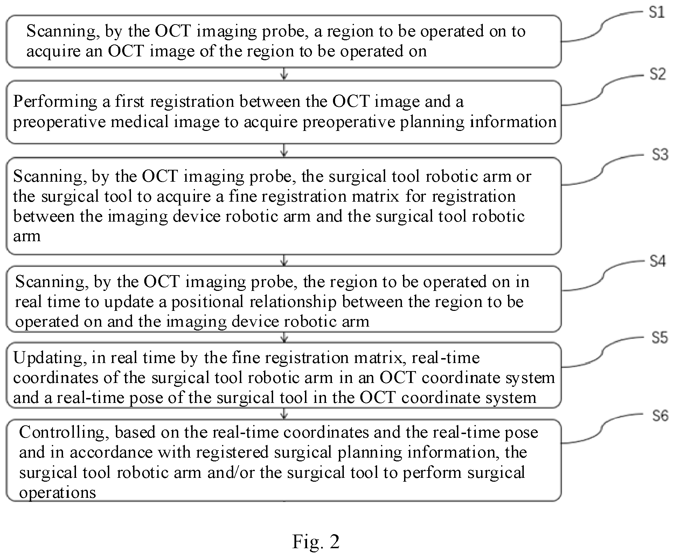

Fig. 2 shows the OCT imaging-based positioning and navigation method for an orthopedic surgical robot 1 according to the present disclosure, which includes the following steps:
S1: scanning, by the OCT imaging probe 3, a region to be operated on to acquire an OCT image of the region to be operated on;
S2: performing a first registration between the OCT image and a preoperative medical image to acquire preoperative planning information;
S3: scanning, by the OCT imaging probe 3, the surgical tool robotic arm 22 or the surgical tool 23 to acquire a fine registration matrix for registration between the imaging device robotic arm 21 and the surgical tool robotic arm 22;
S4: scanning, by the OCT imaging probe 3, the region to be operated on in real time to update a positional relationship between the region to be operated on and the imaging device robotic arm 21;
S5: updating, in real time by the fine registration matrix, real-time coordinates of the surgical tool robotic arm 22 in an OCT coordinate system and a real-time pose of the surgical tool 23 in the OCT coordinate system; and
S6: controlling, based on the real-time coordinates and the real-time pose and in accordance with registered surgical planning information, the surgical tool robotic arm 22 and/or the surgical tool 23 to perform surgical operations. Thus, the method of the present disclosure can achieve a high navigation accuracy of approximately 0.15 mm, and while eliminate intraoperative scanning on the patient by medical imaging device, reduce the radiation exposure received by the patient, and be more conducive to the patient's physical health.

In the step S2, the surgical planning information in the preoperative medical image may be converted into surgical planning information in the OCT coordinate system by a first registration matrix. The first registration matrix is preferably calculated based on a three-dimensional coordinate point cloud of the OCT image and a three-dimensional coordinate point cloud of the medical image, where the preoperative medical image is collected by medical imaging device, and the attending physician responsible for the patient preoperatively can establish the surgical planning information on a three-dimensional model of the preoperative medical image.

Before the step S3, a second registration matrix between the imaging device robotic arm 21 and the surgical tool robotic arm 22 is calculated based on a positional relationship between the imaging device robotic arm 21 and the surgical tool robotic arm 22, where the second registration matrix is calculated based on coordinates of the imaging device robotic arm 21 in a robot coordinate system and coordinates of the surgical tool robotic arm 22 in the robot coordinate system. By the second registration matrix, coordinates of the visual field region of the OCT imaging probe 3 in an imaging device robotic arm 21 coordinate system are transformed into coordinates in a surgical tool robotic arm coordinate system, so as to move the surgical tool robotic arm 22 into the visual field region, where the visual field region is an area near the region to be operated on that can be scanned by the OCT imaging device.

In the step S3, the fine registration matrix is acquired by scanning a first marker 5 that is provided on the surgical tool robotic arm 22 or the surgical tool 23 and is recognizable by the OCT imaging probe 3.

In the step S4, a second marker that is provided in the region to be operated on and is recognizable by the OCT imaging probe 3 is scanned by the OCT imaging probe 3 to acquire real-time coordinates of the region to be operated on in the OCT coordinate system.

In the step S5, real-time coordinates of the surgical tool robotic arm 22 in its surgical tool robotic arm coordinate system are acquired by the surgical tool robotic arm system itself, and a real-time pose of the surgical tool 23 in the surgical tool robotic arm coordinate system is acquired based on a size of the surgical tool 23 and a shape of the surgical tool 23; then, the real-time coordinates and the real-time pose are respectively transformed into the real-time coordinates of the surgical tool robotic arm 22 in the OCT coordinate system and the real-time pose of the surgical tool 23 in the OCT coordinate system by the fine registration matrix.

To reduce the participation of medical staff in orthopedic surgical robot operations and thereby increasing the automation degree of the orthopedic surgical robot 1 implementing the method of the present disclosure, especially to enable the orthopedic surgical robot to automatically move the imaging device robotic arm 21 to the imaging position without medical staff intervention, a capturing apparatus 4 with a same visual field as the OCT imaging probe 3 may be further provided, which is preferably implemented as a remote microscopic device, e.g., a camera. It can identify a marker for marking the surgical area on the surgical drape, e.g., an ArUco marker, so as to acquire original position coordinates of the region to be operated on in the OCT imaging device coordinate system. Based on this, the orthopedic surgical robot automatically moves the imaging device robotic arm 21 to an imaging position directly above and facing the region to be operated on.

Because the region to be operated on is tracked by the OCT imaging device, the pose relationship between the surgical tool 23 and the region to be operated on can be updated in real time. The registration of the positioning and navigation method according to the present disclosure only requires registration between the surgical tool robotic arm/surgical tool coordinate system and the imaging device robotic arm 21 coordinate system, thereby eliminating the registration from the intraoperative medical imaging device coordinate system to the surgical tool robotic arm coordinate system. This simplifies the registration method and reduces the radiation of the surgical object caused by intraoperative use of medical imaging device.

While examples of the present disclosure are provided in the foregoing description, modifications and variations may be made to these examples by those skilled in the art without departing from the scope and spirit of the present disclosure. For example, it should be understood that features of the embodiments herein may be applied to other embodiments described herein. Accordingly, the description is intended to be illustrative rather than restrictive. The present disclosure is defined by the appended claims, and all changes to the present disclosure that come within the meaning and range of equivalency of the claims are to be embraced within their scope.

## Claims

1. An OCT imaging-based positioning and navigation method for an orthopedic surgical robot, the orthopedic surgical robot comprising an OCT imaging device with an OCT imaging probe, an imaging device robotic arm for mounting the OCT imaging device, and at least one surgical tool robotic arm for mounting a surgical tool, wherein the positioning and navigation method comprises the steps of:
S1: scanning, by the OCT imaging probe, a region to be operated on to acquire an OCT image of the region to be operated on;
S2: performing a first registration between the OCT image and a preoperative medical image to acquire preoperative planning information;
S3: scanning, by the OCT imaging probe, the surgical tool robotic arm or the surgical tool to acquire a fine registration matrix for registration between the imaging device robotic arm and the surgical tool robotic arm;
S4: scanning, by the OCT imaging probe, the region to be operated on in real time to update a positional relationship between the region to be operated on and the imaging device robotic arm;
S5: updating, in real time by the fine registration matrix, real-time coordinates of the surgical tool robotic arm in an OCT coordinate system and a real-time pose of the surgical tool in the OCT coordinate system; and
S6: controlling, based on the real-time coordinates and the real-time pose and in accordance with registered surgical planning information, the surgical tool robotic arm and/or the surgical tool to perform surgical operations.

2. The positioning and navigation method according to claim 1, wherein in the step S2, the surgical planning information in the preoperative medical image is converted into surgical planning information in the OCT coordinate system by a first registration matrix.

3. The positioning and navigation method according to claim 2, wherein the first registration matrix is calculated based on a three-dimensional coordinate point cloud of the OCT image and a three-dimensional coordinate point cloud of the medical image.

4. The positioning and navigation method according to claim 1 or 2, wherein before the step S3, a second registration matrix between the imaging device robotic arm and the surgical tool robotic arm is calculated based on a positional relationship between the imaging device robotic arm and the surgical tool robotic arm.

5. The positioning and navigation method according to claim 4, wherein the second registration matrix is calculated based on coordinates of the imaging device robotic arm in a robot coordinate system and coordinates of the surgical tool robotic arm in the robot coordinate system.

6. The positioning and navigation method according to claim 4, wherein coordinates of visual field region of the OCT imaging probe in an imaging device robotic arm coordinate system are transformed into coordinates in a surgical tool robotic arm coordinate system by the second registration matrix, so as to move the surgical tool robotic arm into the visual field region.

7. The positioning and navigation method according to claim 6, wherein the visual field region is an area near the region to be operated on that is scanned by the OCT imaging device.

8. The positioning and navigation method according to claim 1 or 2, wherein in the step S3, the fine registration matrix is acquired by scanning a first marker that is provided on the surgical tool robotic arm or the surgical tool and is recognizable by the OCT imaging probe.

9. The positioning and navigation method according to claim 1 or 2, wherein in the step S4, a second marker that is provided in the region to be operated on and is recognizable by the OCT imaging probe is scanned by the OCT imaging probe to acquire real-time coordinates of the region to be operated on in the OCT coordinate system.

10. The positioning and navigation method according to claim 1 or 2, wherein in the step S5, real-time coordinates of the surgical tool robotic arm in a surgical tool robotic arm coordinate system are acquired by the surgical tool robotic arm system itself, and a real-time pose of the surgical tool in the surgical tool robotic arm coordinate system is acquired based on a size of the surgical tool and a shape of the surgical tool; and, the real-time coordinates of the surgical tool robotic arm in the surgical tool robotic arm coordinate system and the real-time pose of the surgical tool in the surgical tool robotic arm coordinate system are respectively transformed into the real-time coordinates of the surgical tool robotic arm in the OCT coordinate system and the real-time pose of the surgical tool in the OCT coordinate system by the fine registration matrix.

11. The positioning and navigation method according to claim 1 or 2, wherein a capturing apparatus with a same visual field as the OCT imaging probe is provided, and a marker for marking the region to be operated on that is recognizable by the capturing apparatus is identified by the capturing apparatus to acquire original position coordinates of the region to be operated on in the OCT imaging device coordinate system, so as to calculate an imaging position directly above and facing the region to be operated on based on the original position coordinates, and move the imaging device robotic arm to the imaging position.
